# EUROPEAN PATENT SPECIFICATION

(11) **EP 4 281 454 B1**
(45) Date of publication and mention of the grant of the patent: **05.03.2025**
(21) Application number: 22702203.5
(22) Date of filing: 20.01.2022
(51) Int. Cl.: C07D 487/04

(54) **PROCESS FOR MAKING IBRUTINIB**
VERFAHREN ZUR HERSTELLUNG VON IBRUTINIB
PROCÉDÉ DE PRÉPARATION D'IBRUTINIB

(30) Priority: 21.01.2021 EP 21152830
(43) Date of publication of application: 29.11.2023
(73) Proprietor: Synthon B.V., 6545 CM Nijmegen (NL)
(72) Inventor: MITAS, Petr, 67817 Blansko (CZ); VYKLICKY, Libor, 67817 Blansko (CZ); BARTL, Jiri, 67817 Blansko (CZ); SMEKAL, Oldrich, 67817 Blansko (CZ)
(74) Representative: Dobsík, Martin
(86) International application number: PCT/EP2022/051250
(87) International publication number: WO 2022/157250

(56) References cited:
- WO-A1-2016/066673
- WO-A2-2008/039218
- ZOU YI ET AL: "Structure-based discovery of novel 4,5,6-trisubstituted pyrimidines as potent covalent Bruton's tyrosine kinase inhibitors", BIOORGANIC & MEDICINAL CHEMISTRY LETTERS, ELSEVIER, AMSTERDAM, NL, vol. 26, no. 13, 7 May 2016 (2016-05-07), pages 3052 - 3059, XP029568924, ISSN: 0960-894X, DOI: 10.1016/J.BMCL.2016.05.014
- ZOU YI ET AL: "Supplementary Data Structure-based discovery of novel 4,5,6-trisubstituted pyrimidines as potent covalent Bruton's tyrosine kinase inhibitors Table of contents", 7 May 2016 (2016-05-07), pages S1 - S21, XP055903967, Retrieved from the Internet <URL:https://ars.els-cdn.com/content/image/1-s2.0-S0960894X1630498X-mmc1.docx> [retrieved on 20220322]

## Description

### BACKGROUND OF THE INVENTION

The present invention relates to an improved process for making Ibrutinib.

### BACKGROUND OF THE INVENTION

Ibrutinib, chemically 1-[3(R)-[4-Amino-3-(4-phenoxyphenyl)-1H-pyrazolo[3,4-d]pyrimidin-1-yl]piperidin-1-yl]-2-propen-1-one, compound of Formula (1): 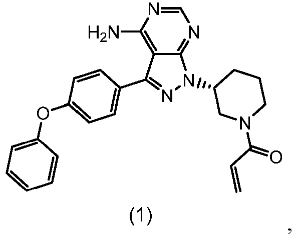 is a pharmaceutically active compound acting as an oral Bruton's tyrosine kinase (BTK) inhibitor. Ibrutinib is used as a medicament for the treatment of patients with mantle cell lymphoma and for the treatment of chronic lymphocytic leukemia. Ibrutinib is available, e.g., under the brand name Imbuvica^{®}.

Ibrutinib was disclosed in WO2008039218 by Pharmacyclics. There are several applications and articles describing a process for preparation of Ibrutinib, for example WO2008039218 by Pharmacyclics or WO2016050422 by Ratiopharm or WO2016170545 by MSN or WO2017134684 by Natco or WO2016115356A1 by Janssen or WO2016066673 by Sandoz or Zou Yi et al., Bioorg. Med. Chem Lett. 26 (13), 2016, pages 3052-3059, and Zou Yi et al.: "Supplementary Data Structure-based discovery of novel 4,5,6-trisubstituted pyrimidines as potent covalent Bruton's tyrosine kinase inhibitors Table of contents", (2016-05-07), pages S1-S21. The processes described in the prior art do not provide Ibrutinib in sufficient purity or contain column purification in purification steps of either Ibritinib or used intermediates. Column purification is not suitable to be used on an industrial scale production.

Hence, there is a need for an improved process for preparing of Ibrutinib. In particular, it would be desirable to have a process which is efficient in terms of yield and chemical purity, cost effective in terms of reagents and reaction conditions, and which is applicable on an industrial scale.

There are also several solid forms described in the prior art, for example solid forms A and C described in WO2013184572 by Pharmacyclics. The application further describes several solvates of Ibrutinib.

### BRIEF DESCRIPTION OF THE INVENTION

The presented invention relates to a process for preparation of Ibrutinib, compound of Formula 1 or a salt thereof, 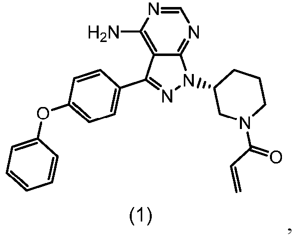 the process comprising:
a. Reacting compound of Formula (2) with compound of Formula (3) in a presence of Lewis acid to provide compound of Formula (4), 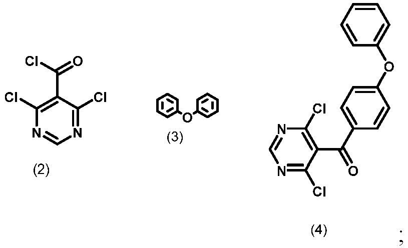
b. Contacting compound of Formula (4) with toluene;
c. Isolating a solid form of compound of Formula (4);
d. Transforming compound od Formula (4) into Ibrutinib.

### BRIEF DESCRIPTION OF THE DRAWINGS

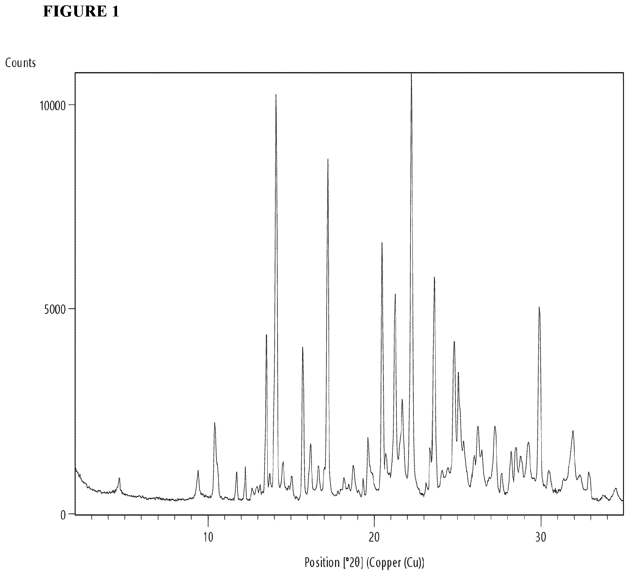

Figure 1 depicts the X-Ray Powder Diffractogram (XRPD) of solid form of compound of Formula (4), Form 1, prepared by the process described in Example 1.

### DETAILED DESCRIPTION OF THE INVENTION

The presented invention relates to a process for preparation of Ibrutinib, compound of Formula 1, or a salt thereof, 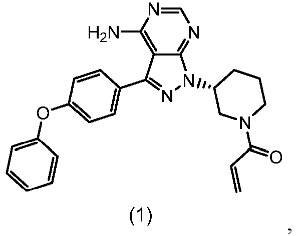 the process comprising:
a. Reacting compound of Formula (2) with compound of Formula (3) in a presence of Lewis acid to provide compound of Formula (4) 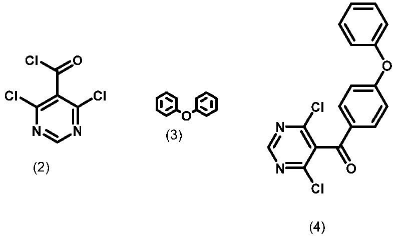
b. Contacting compound of Formula (4) with toluene;
c. Isolating a crystalline form of compound of Formula (4);
d. transforming compound od Formula (4) into Ibrutinib.

The reaction between compound of Formula (2) and Formula (3) can be performed in a suitable solvent for example 2-methyl tetrahydrofurane or tetrahydrofurane or dichloromethane, preferably dichloromethane is used. In case dichloromethane is not used as a solvent, it is added to the mixture. The reaction is performed in presence of a Lewis acid. The molar ratio between dichloromethane and the compound of Formula (2) can be between 1:0.7 and 1:1.2, preferably it is between 1:0.7 and 1:0.9. As a Lewis acid AlCl₃ or FeCl₃ or BF₃.Et₂O can be used. The molar ratio between the Lewis acid and the compound of formula (2) can be between 1:1 and 1:1.4, preferably between 1:1 and 1:1.2. The molar ratio between compound of Formula (2) and compound of Formula (3) can be between 1:1 and 1:1.2, preferably it is 1:1.1. Compound of Formula (2) is dissolved in the solvent, preferably dichloromethane. The concentration of compound of formula (2) in the solvent can be between 2 g/g and 6 g/g. In the preferred embodiment when dichloromethane is used as a solvent, the concentration of the compound of Formula (2) in dichloromethane can be between 3 g/g and 4 g/g, preferably it is between 3.0 g/g and 3.4 g/g. The mixture is heated to a temperature between 30°C and 40°C and 2/3 (molar ratio with respect to total amount of the Lewis acid) of Lewis acid is added and dissolved in the mixture. Then compound of Formula (3) is added in the course of between 10 and 30 minutes. Then remaining 1/3 (molar ratio with respect to total amount of the Lewis acid) of the Lewis acid is added to the mixture. The mixture is then stirred at a temperature between 30°C and 40°C for between 30 and 60 minutes. Then the mixture is heated to between 65°C and 70°C and the mixture is stirred at this temperature for between 60 and 120 minutes. The reaction progress can be monitored by any suitable technique, for example HPLC or GC. After the reaction is finished, the reaction mixture is cooled to a temperature between 35°C and 40°C. The reaction mixture is added into an acetate such as methyl acetate or ethyl acetate. The reaction mixture is added in several portion, for example in 1 or 2 or 3 or 4 or 5 or 6 portion, preferably it is added dropwise. The mixture is added into water in the course of between 40 and 90 minutes. The mixture is added in several portion, for example in 1 or 2 or 3 or 4 or 5 or 6 portion, preferably it is added dropwise. The weight ratio between compound of Formula (2) and the acetate can be between 1:1 and 1:2, preferably it is between 1:1 and 1:1.3. The weight ratio between the acetate and water can be between 1:2 and 1:3, preferably it is between 1:2 and 1:2.5. The resulting mixture is mixed with toluene. The weight ratio between toluene and the acetate can be between 1:1.2 and 1:2, preferably it is between 1:1.2 and 1:1.5. The phases are separated and the water phase is extracted twice with toluene. The weight ratio between toluene and the acetate can be between 1:0.5 and 1:1, preferably it is between 1:0.6 and 1:0.8. Combined organic phases are evaporated to between 45% and 55%, preferably to 50% (acetate and a part of toluene are distilled off from the mixture) of the original volume to obtain a suspension. The mixture is cooled to a temperature between (-5°C) and 0°C and stirred at this temperature for between 60 and 120 minutes. The solid mass is filtered off and optionally washed with toluene and dried.

Obtained solid compound of Formula (4), Form 1, can be characterized by XRPD pattern having 2θ values 14.1°, 17.2°, 20.5° and 22.3° degrees 2 theta (± 0.2 degrees 2 theta). The solid form can be also characterized by XRPD pattern having 2θ values 13.5°, 14.1°, 15.7°, 17.2°, 20.5°, 21.3° and 22.3° degrees 2 theta (± 0.2 degrees 2 theta). The solid form can be further characterized by XRPD pattern described in the following table:

| **Angle (2-Theta °)** | **Intensity %** | **Angle (2-Theta °)** | **Intensity %** |
|---|---|---|---|
| 4.7 | 8.0 | 20.7 | 13.6 |
| 9.4 | 10.1 | 21.3 | 50.7 |
| 10.4 | 20.9 | 21.7 | 26.1 |
| 10.6 | 11.7 | 22.3 | 100.0 |
| 11.1 | 3.7 | 23.1 | 7.1 |
| 11.7 | 9.8 | 23.4 | 15.2 |
| 12.2 | 10.8 | 23.6 | 54.7 |
| 12.7 | 5.9 | 24.1 | 9.8 |
| 12.9 | 6.0 | 24.4 | 10.4 |
| 13.1 | 6.6 | 24.8 | 39.3 |
| 13.5 | 43.5 | 25.1 | 32.7 |
| 13.8 | 9.4 | 25.4 | 16.4 |
| 14.1 | 97.3 | 26.0 | 13.2 |
| 14.5 | 11.8 | 26.2 | 19.7 |
| 15.0 | 8.4 | 26.5 | 14.4 |
| 15.7 | 39.0 | 26.9 | 8.3 |
| 16.2 | 15.8 | 27.2 | 20.0 |
| 16.6 | 10.7 | 27.7 | 9.4 |
| 17.2 | 81.5 | 28.2 | 13.9 |
| 17.8 | 5.1 | 28.5 | 15.1 |
| 18.0 | 5.6 | 28.8 | 13.1 |
| 18.2 | 8.1 | 29.3 | 16.2 |
| 18.5 | 7.1 | 29.9 | 46.7 |
| 18.8 | 11.1 | 30.5 | 9.9 |
| 19.1 | 5.5 | 31.4 | 7.9 |
| 19.3 | 7.9 | 31.9 | 19.2 |
| 19.6 | 17.6 | 32.4 | 8.8 |
| 19.9 | 9.1 | 32.9 | 9.5 |
| 20.5 | 61.4 | | |

The solid Form 1 can be also characterized by XRPD pattern depicted in Figure 1.

The compound of Formula (4) can be purified by a process comprising:
a. Dissolving compound of Formula (4) in toluene;
b. Isolating the solid form of compound of Formula (4).

The weight ratio between compound of Formula (4) and toluene can be between 1:0.9 and 1:1. Compound of Formula (4) can be dissolved in toluene at a temperature between 65°C and 75°C. The mixture is cooled to between -15°C and -5°C and stirred at this temperature for between 3 and 6 hours. Obtained suspension is filtered off and optionally dried to obtain the solid Form 1 of compound of Formula (4) in a yield between 90% and 95%.

The compound of Formula (2) can be prepared by a process described in the prior art or by a process comprising:
a. Contacting compound of Formula (9) with oxalyl chloride or thionyl chloride in 2-methyl tetrahydrofurane in a presence of dimethylformamide, 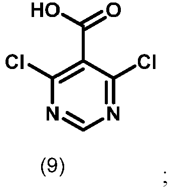
b. evaporating the reaction mixture;
c. dissolving the rest in heptane;
d. isolating compound of Formula (2).

Contrary to the solvents described in prior art 2-methyl tetrahydrofurane (MeTHF) improves the purity of obtained compound of Formula (2). The concentration of compound of Formula (9) in 2-methyl tetrahydrofurane can be between 0.9 g/g and 1.3 g/g. The molar ratio between the compound of Formula (9) and dimethylformamide (DMF) can be between 1:0.009 and 1:0.013. The molar ratio between oxalyl chloride or thionyl chloride and the compound of Formula (9) can be between 1.2:1 and 1.8:1, preferably it is between 1.3:1 and 1.5:1. Compound of Formula (9) is dissolved in 2-methyltetrahydrofurane under a protective atmosphere, for example nitrogen or argon. Dimethylformamide is added to the mixture. The mixture is cooled to a temperature between 15°C and 20°C. Oxalyl chloride or thionyl chloride is added to the mixture in the course of between 45 and 120 minutes. The mixture is stirred for between 10 and 30 minutes. The reaction progress can be monitored by any suitable technique, for example HPLC. After the reaction is finished, the mixture is concentrated at 50-60°C and under vacuum (100 mbar). Obtained residue is cooled to room temperature (20-25°C) and contacted with heptane. The concentration of compound of Formula (2) in heptane can be between 0.7 g/g and 1.2 g/g, preferably it is between 0.8 g/g and 1.1 g/g. Resulting mixture is stirred for between 10 and 30 minutes at 20-25°C, filtered and the filtrate is evaporated to provide compound of Formula (2) in excellent yield and purity.

Compound of Formula (4) can be transformed into Ibrutinib, compound of Formula (1), by a process described in the prior art or by a process comprising:
i. Reacting compound of Formula (4) with ammonia to obtain a mixture;
ii. Reacting the mixture with hydrazine to obtain compound of Formula (5),

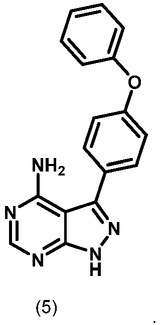

Ammonia in step i. can be either gaseous ammonia or a solution of ammonia can be used. Preferably water solution of ammonia is used. Water solution of ammonia can be 15% or 20% or 25% solution of ammonia in water, preferably 25% solution is used. The reaction step i. can be optionally performed in a suitable solvent, for example dioxane or toluene, preferably dioxane is used. Use of dioxane improves the processability (filterability) of prepared compound of Formula (5). The concentration of compound of Formula (4) in the solvent can be between 0.3 g/g and 0.7 g/g, preferably it is between 0.4 g/g and 0.6 g/g. The molar ratio between compound of Formula (4) and ammonia can be between 1:3 and 1:5, preferably it is between 1:3.1 and 1:3.7. Compound of Formula (4) is dissolved in the solvent. The mixture is heated to a temperature between 50°C and 80°C and ammonia is added. The mixture is stirred for between 1 and 5 hours. The reaction progress can be monitored by any suitable technique, for example HPLC. After the reaction is finished, the reaction mixture is heated to a temperature between 80°C and 95 °C and to the mixture hydrazine is added. Hydrazine can be in a form of a salt, for example hydrazine hydrochloride or in a form of a hydrate. Hydrazine hydrate is preferably used. Hydrazine can be added as a solid or can be used in a solution in a suitable solvent. The reaction mixture is stirred for between 1 and 5 hours. The reaction progress can be monitored by any suitable technique, for example HPLC. After the reaction is finished, to the mixture water is added at an elevated (80 - 95°C) temperature. The ratio (wt:wt, weight : weight) between water and the solvent used in step i. can be between 1.8:1 and 2.5:1. The mixture is then cooled to a temperature between 15°C and 20°C. Obtained solid is filtered off and optionally washed with ethyl acetate and water and dried. Stirring of obtained compound of Formula (5) in a mixture of dioxane and water (weight ratio 1:3) to improve purity of obtained compound of formula (5) (weight ratio compound of Formula (5):dioxane:water was 0.5:1:3) can be used.

Compound of Formula (5) can be transformed into Ibrutinib, compound of Formula (1) by a process described in the prior art or by a process comprising
i. Reacting compound of Formula (5) with compound of Formula (6) in a suitable solvent to provide compound of Formula (7), 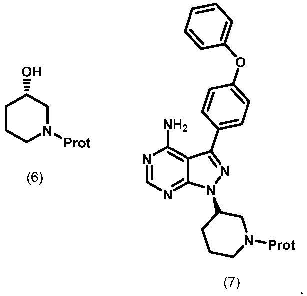 Prot is an amine protecting group, preferably Boc (tert-butoxy carbonyl) group
ii. Deprotecting compound of Formula (7) to provide compound of Formula (8) or a salt thereof, 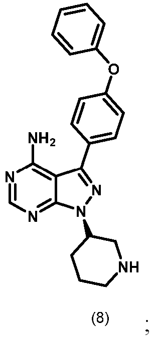
iii. Transforming compound of Formula (8) or a salt thereof into Ibrutinib

The step i. is performed in a presence of triphenylphospine and an azodicarboxylate such as diisopropyl azodicarboxylate or diethyl azodicarboxylate or di-tert-butyl azodicarboxylate or dibenzyl azodicarboxylate. The reaction is performed in a suitable solvent such as 2-methyl tetrahydrofurane or tetrahydrofurane or toluene or dimethylformamide, preferably 2-methyltetrahydrofurane (MeTHF) is used. The molar ratio between compound of Formula (5) and triphenylphosphine can be between 1:2 and 1:4, preferably it is between 1:2 and 1:3. The molar ratio between compound of Formula (5) and the azodicarboxylate, preferably diisopropyl azodicarboxylate can be between 1:2 and 1:3, preferably it is between 1:2.2 and 1:2.6. The molar ratio between compound of Formula (5) and compound of Formula (6) can be between 1:2 and 1:3, preferably it is between 1:2 and 1:2.5. Compound of Formula (5), triphenylphosphine and compound of Formula (6) are mixed with the solvent. The concentration of compound of Formula (5) in the solvent can be between 0.1 g/g and 0.3 g/g, preferably it is between 0.1 and 0.2 g/g. The mixture is heated to a temperature between 35°C and 50°C and the azodicarboxylate, preferably diisopropyl azodicarboxylate in a suitable solvent is added. The solvent can be the same solvent as was used for dissolving compound of Formula (5) or it can be different. Preferably it is 2-methyl tetrahydrofurane. The concentration of the azodicarboxylate, preferably diisopropyl azodicarboxylate in the solvent can be between 0.8 g/g and 2 g/g, preferably it is between 1 g/g and 1.5 g/g. The azodicarboxylate, preferably diisopropyl azodicarboxylate is preferably added in the course of between 45 and 120 minutes. The mixture can be then stirred for between 10 and 60 minutes. The reaction progress can be monitored by any suitable technique, for example HPLC. After the reaction is finished, compound of Formula (7) is deprotected by a suitable process, for example using an acid (for examle HCl or other strong acid such as trifluoroacetic acid) or hydrogenation deprotection using hydrogen in a presence of a suitable catalyst. Deprotection using HCl, more preferably aqueous solution of HCl, is preferably used. The aqueous solution is preferably concentrated HCl (35-36%) water solution. The molar ratio between the acid and the compound of Formula (5) can be between 5:1 and 8:1, preferably it is between 6:1 and 7:1. The deprotection step can be performed at an elevated temperature, for example between 40°C and 60°C. The acid is added to the mixture prepared in the previous step and the mixture is stirred for between 60 and 120 minutes. The reaction progress can be monitored by any suitable technique, for example HPLC. After the reaction is finished, layers are separated. In comparison with processes described in the prior art, using the described process comprising reaction step i. in 2-methyl tetrahydrofurane and subsequent deprotection using aqueous solution of HCl improves the purity of obtained compound of Formula (8) with respect to reaction side-products mostly originated from triphenylphosphine or the azodicarboxylate. The side products remain in organic phase, compound of Formula (8) in form of a salt, preferably HCl salt, remains in water phase and is separated from the impurities during phases separation.

The water layer is heated to between 65°C and 75°C. To the water layer 2-propanol is added. The weight ratio between 2-propanol and the compound of Formula (5) can be between 1:10 and 1:20, preferably it is between 1:12 and 1:15. The mixture is then stirred for between 30 and 60 minutes. The mixture is then cooled to between 0°C and 15°C and stirred at this temperature for between 1 and 5 hours. Obtained solid compound of Formula (8) or a salt thereof is filtered off, optionally washed with 2-propanol and dried.

In case that compound of Formula (8) is obtained in a form of a salt it can be transformed into free form of compound of Formula (8) by contacting with a base, preferably in a mixture of water and 2-propanol. The molar ratio between the salt of compound of Formula (8) and the base can be between 1:2 and 1:2.5. The salt of compound of Formula (8) is dissolved in water. The concentration of the salt of compound of Formula (8) in water can be between 0.15 g/g and 0.7 g/g, preferably it is between 0.25 g/g and 0.4 g/g. The mixture is heated to a temperature between 40°C and 60°C. To the mixture 2-propanol is added. The weight ratio between water and 2-propanol can be between 1.5:1 and 3:1, preferably it is between 1.9:1 and 2.2:1. To the mixture a base, preferably in a form of water solution is added. The base is preferably a hydroxide (such as sodium hydroxide or potassium hydroxide). The concentration of the base in water can be between 0.1 and 0.7 g/g, preferably it is between 0.15 and 0.3 g/g. The base is preferably added in the course of between 30 and 90 minutes. Obtained mixture is then stirred for between 30 and 90 minutes and then cooled to between 5 and 10°C. Solid compound of Formula (8) is filtered off, optionally washed with water and dried.

Disclosed process comprising preparation of a salt of compound of Formula (8) by contacting compound of Formula (8) with an acid and subsequent transformation to the compound of Formula (8) using a base can be used for further purification of compound of Formula (8).

Compound of Formula (8) can be transferred into Ibrutinib, compound of Formula (1) by a process disclosed in the prior art using acrylic acid or acrylloyl chloride or 3-chloropropionyl chloride or by a process comprising:
i. Reacting compound of Formula (8) with acryloyl chloride in 2-methyl tetrahydrofurane;
ii. Filtering the mixture;
iii. Adding an acid into the filtrate;
iv. Contacting the filtrate with active carbon;
v. Isolating Ibrutinib.

The molar ratio between compound of Formula (8) and acryloyl chloride can be between 1:1 and 1:1.3. The reaction is performed in a presence of a base for example an amine (such as diethylamine or diisopropylethylamine or triethylamine or 1,8-diazabicyklo(5.4.0)undec-7-en (DBU)). The molar ratio between the base and the compound of Formula (8) can be between 1:1 and 1.3:1. Compound of Formula (8) is dissolved in 2-methyl tetrahydrofurane (MeTHF) and the base is added. The concentration of compound of Formula (8) in 2-methyl tetrahydrofurane can be between 0.1 g/g and 0.3 g/g, preferably it is between 0.11 g/g and 0.16 g/g. The mixture is cooled to (-10)°C-(-20°C). Acryloyl chloride is added to the mixture in the course of between 45 and 120 minutes. Acryloyl chloride is preferably used in a form of a solution in a suitable solvent, preferably in 2-methyl tetrahydrofurane. The concentration of acryloyl chloride in the solvent can be between 0.07 g/g and 0.12 g/g. The mixture was then stirred for between 10 and 60 minutes. The reaction progress can be monitored by any suitable technique, for example HPLC. After the reaction is finished, the mixture is heated to between 10°C and 15°C Solid mass is filtered off and washed with the suitable solvent. To the filtrate an acid for example HCl, preferably 35% water solution of HCl is added. The weight ratio between the added acid and the compound of Formula (8) can be between 1:63 and 1:69, preferably it is between 1:65 and 1:67. The mixture can be optionally heated to a temperature between 40°C and 50°C and active carbon is added. The ratio (wt:wt) between compound of Formula (8) and the active carbon can be between 15:1 and 25:1, preferably it is 20:1. The mixture is then stirred for between 30 and 90 minutes and filtered off. The filtrate is then concentrated to provide Ibrutinib, compound of Formula (1). Using the described procedure improves the purity of obtained Ibrutinib in comparison with procedures disclosed in the prior art. Obtained Ibrutinib can be transformed into a solvate form, such as toluene solvate or other solvates disclosed in the prior art or by the process described in Example 6 or by the process described later. Ibrutinib can also be transformed into polymorphic forms C or A or other polymorphic forms disclosed in the prior art (for example in WO2013184572) or by a process disclosed in Example 5 or 7 or by the processes described later. Ibrutinib can be transformed into polymorphic form C (disclosed in WO2013184572) by a process comprising:
i. Dissolving Ibrutinib in a mixture of 2-methyl tetrahydrofurane and methanol;
ii. Isolating Ibrutinib form C.

The volume ratio 2-methyl tetrahydrofurane: methanol can be between 1:7 and 1:12. The concentration of Ibrutinib in the mixture can be between 0.2 g/g and 0.4 g/g. Ibrutinib is contacted with the mixture 2-methyl tetrahydrofurane:methanol and the mixture is heated to between 40°C and 55°C to obtain a solution. The solution is cooled to a temperature between 20°C and 25°C and stirred at this temperature for between 3 and 6 hours. The obtained suspension was cooled to a temperature between 0 and 5°C and stirred at this temperature for between 2 and 5 hours. Obtained solid is filtered off, washed with methanol and dried to provide Ibrutinib in crystalline Form C (disclosed in WO2013184572).

Ibrutinib, preferably in crystalline form C, can be contacted with methyl tert-butyl ether (MTBE) to provide Ibrutinib crystalline form A (disclosed in WO2013184572). Concentration of Ibrutinib in methyl tert-butyl ether can be between 0.15 g/g and 0.25 g/g. Ibrutinib is mixed with methyl tert-butyl ether, the mixture is stirred at a temperature between 35°C and 45°C for between 2 and 5 hours. The mixture is then cooled to a temperature between 20°C and 25°C and filtered off. Obtained solid can be washed with methyl tert-butyl ether and dried to provide Ibrutinib in crystalline form A.

Ibrutinib can be also transformed into a toluene solvate by a process comprising:
i. Dissolving Ibrutinib in a mixture comprising 2-methyl tetrahydrofurane and toluene;
ii. Isolateng Ibrutinib toluene solvate.

The volume ratio 2-methyl tetrahydrofurane:toluene can be between 1:1 and 1:3, preferably it is 1:2. Concentration of Ibrutinib in the mixture can be between 0.08 g/g and 0.2 g/g, preferably it is between 0.1 g/g and 0.15 g/g. Ibrutinib is dissolved in the mixture at a temperature between 50°C and 65°C. The solution is cooled to a temperature between 0°C and 5°C and stirred at this temperature for between 10 and 60 minutes. Obtained solid is filtered off and washed with toluene to provide toluene solvate of Ibrutinib.

The invention will be further described with reference to the following examples.

### EXAMPLES

XRPD spectrum was obtained using the following measurement conditions:
Panalytical Empyrean diffractometer with Θ/2Θ geometry (transmition mode), equipped with a PixCell 3D detector;

| | |
|---|---|
| Start angle (2θ): | 2.0° |
| End angle (2θ): | 35.0° |
| Step size: | 0.026° |
| Scan speed: | 0.0955 °/seconds |
| Radiation type: | Cu |
| Radiation wavelengths: | 1.5406Å (Kα1), primary monochromator used |

| | |
|---|---|
| Divergence slit: | 1/2° |
| Antiscatter slit: | 1/2° |
| Soller slit: | 0.02 rad |
| Detector slit: | 7.5 mm |
| Rotation speed: | 30 rpm |

### Example 1: Preparation of compound of Formula (4)

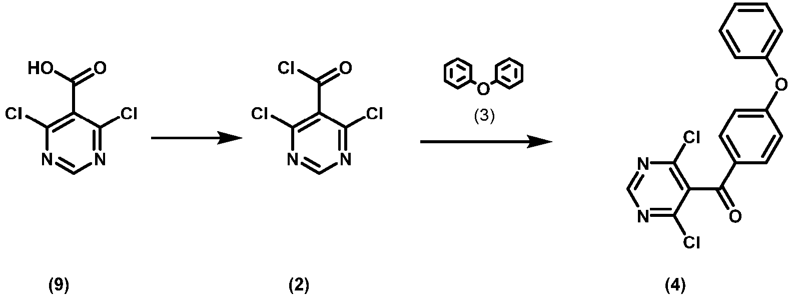

150 g of 4,6-dichloropyrimidine-5-carboxylic acid (compound of Formula (9)) was dissolved in 150 g of 2-methyl tetrahydrofurane (MeTHF) under nitrogen atmosphere and 0.65 g of dimethylformamide (DMF) was added. The mixture was cooled to 20° C and 127.5 g of oxalyl chloride was added dropwise in the course of 60 minutes. The mixture was stirred for 10 minutes. The mixture was evaporated (100 mbar, 50 - 60° C). Obtained residue was cooled to 20 - 25° C and to the mixture 180 g of n-heptane is added. Obtained mixture was stirred for 10 minutes at 20 - 25°C. The mixture was then filtered, washed with 30 g of n-heptane. The mixture was evaporated (100 mbar, 50 - 60° C) to provide 160 g of compound of Formula (2) as oily liquid in 97.35% yield and 98.21% purity (HPLC IN).

Obtained compound of Formula (2) was dissolved in 50 g of dichloromethane. To the mixture 72 g of AlCl₃ was added in two parts (2x36 g). The mixture was heated to 30°C - 40°C to form a solution. 146 g of diphenylether (compound of Formula (3)) was added in the course of 15 minutes at 30 - 40° C. To the mixture 36 g of AlCl₃ was added. The mixture was stirred at 30 - 40° C for 30 minutes and then for 90 min at 65 - 70° C. The mixture was cooled to 40°C. The mixture was dropped into 150 g of ethyl acetate. The mixture was then dropped into 300 g of water in the course of 50 minutes. Obtained suspension was extracted with 200 g of toluene at 40°C, layers were separated and the water phase was extracted two times with 100 g of toluene at 40° C. The combined organic solutions were evaporated to final weight 550 g and the mixture was cooled to 0 - (-5° C) in the course of 90 minutes. The solid was filtered off at 0 - (-5° C), washed with 2x25 g of cooled (0°C) toluene and vacuum dried (100 mbar, 65° C, 5 hours) to provide 163.8 g (yield 61.05%, purity 99.05% (HPLC IN)) of compound of Formula (4). XRPD pattern of obtained solid corresponds to XRPD pattern depicted in Figure 1.

### Example 2: Preparation of compound of Formula (5)

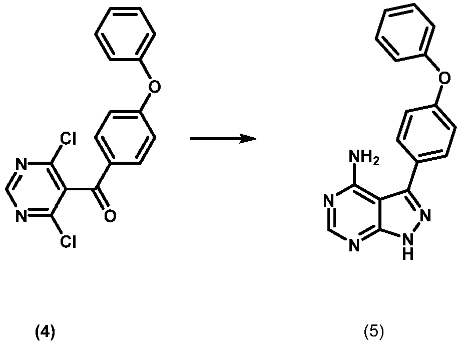

300 g of compound of Formula (4) was dissolved in 600 g of dioxane and the mixture was heated to 60 - 65° C. To the mixture 210 g of aqueous ammonia (25% solution) was added in the course of 15 minutes. The mixture was stirred for 1 hour. The mixture was heated to 80° C and 150 g of Hydrazine hydrate was added. The mixture was stirred for 60 minutes. To the mixture 1200 g of water was added in the course of 1 hour at 80° C. The mixture was cooled to 10 - 15° C in the course of 2 hours. The solid mass was filtered off at 10 - 15° C, washed with 100 g of ethyl acetate, 200 g of water and 100 g of ethyl acetate and vacuum dried (100 mbar, 65 °C, 3hours) to provide 249.70 g of compound of Formula (5) (yield 94.72%, purity 99.41%, (HPLC IN)).

### Example 3: Preparation of compound of Formula (8)

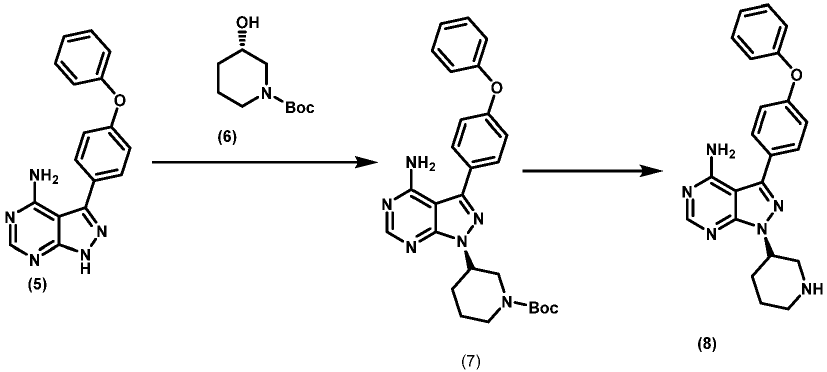

100 g of compound of Formula (5) were mixed with 220 g of Triphenylphosphine, 145 g of compound of Formula (6) and 650 g of 2-methyl tetrahydrofurane (MeTHF). The mixture was heated to 40° C. 165 g of Diisopropyl azodicarboxylate (DIAD) in 160 g of MeTHF was added in the course of 60 minutes and solution was stirred for next 10 minutes.

The mixture was heated to 50° C and 200 g of concentrated (35-36%) Hydrochloric acid water solution was added. The mixture was stirred for 90 minutes. The layers were separated at (40 - 50° C) and water layer was mixed with 1300 g of 2-propanol at 70° C. 2-propanol was added in the course of 30 minutes. The suspension was cooled to 10 - 15 ° C in the course of 2.5 hours. The solid mass was filtered off at 0 - 5° C, washed with 200 g of cooled (5°C) 2-propanol to provide 179 g of compound of Formula (8) in a form of 2.HCl (dihydrochloride) salt.

Obtained solid compound of Formula (8).2HCl was mixed with 566 g of water. The mixture was heated to 50° C. To the mixture 283 g of 2-propanol was added. To the mixture 28.32 g of sodium hydroxide in 142 g of water was added in the course of 45 minutes. The suspension was stirred at 50° C for next 30 minutes. The suspension was cooled to 15 - 20° C in the course of 1.5 hour. The solid mass was filtered off at 5 - 10° C, washed with 400 g of water and vacuum dried (100 mbar, 65° C, 5hours) to provide 103g of compound of formula (8) (yield 80.84%, purity 99.88% (HPLC IN)).

### Example 4: Preparation of Ibrutinib, compound of Formula (1)

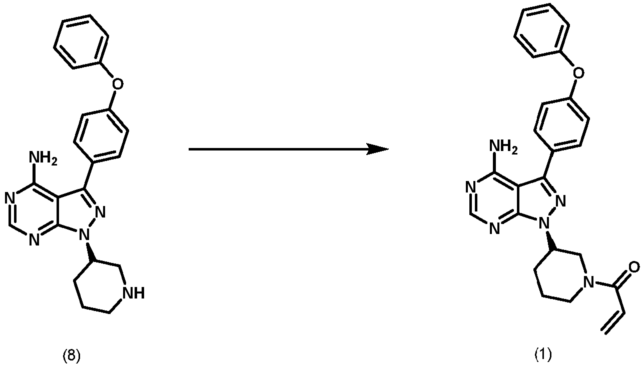

Under nitrogen protection, 20 g of compound of Formula (8) was mixed with 150 g of 2-methyl tetrahydrofurane (MeTHF) and 6.8g of diisopropyl ethylamine (DIPEA). The suspension was cooled down to (-11)-(-13) °C and a solution of 4.7 g of acryloyl chloride in 50g of MeTHF was slowly added dropwise (in the course of 1 hour). The suspension was stirred at -12 °C next 10 minutes. The mixture was filtered at 10 - 15° C and the filter cake was washed with 2x5g of MeTHF.

The filtrate was mixed with 0.30 g of HCl (35-36%) at 25° C. The mixture was heated to 45° C and mixed with 4g of active carbon. The mixture was heated to 50°C and stirred for 0.5 hour. The mixture was filtered at 40°C. The filtrate was concentrated under low pressure (40kPa, 20 - 22° C, 0.5hr) to provide 27.18 g of solid mass in a form of a foam.

The solid was mixed with a mixture of 7g of MeTHF and 80g of methanol and the mixture was heated to 45° C to obtain a solution. The solution was cooled to 25° C in the course of 0.5 hour. The mixture was stirred at 25° C for 3 hours. Obtained suspension was cooled to 5 - 0° C and stirred for next 2 hours. Obtained solid mass was filtered off, washed with 2x5g of cold (0°C) methanol and dried on filter for 0.5 hour and then at 20-25°C for 1 hour to provide 17.95g of compound of Formula (1). (yield 79%, purity 99.7% (HPLC IN)).

### Example 5: Preparation of polymorphic form A of compound of Formula (1)

3g of compound of Formula (1) were suspended in 15g of methyl tert-butyl ether (MTBE) under nitrogen atmosphere and the suspension was stirred at 40° C for 2 hours. The suspension was cooled to room temperature (20-25°C). Obtained solid was filtered off, washed with 2x2g of MTBE and dried on filter for 0.5 hour to provide 2.9g (yield 97%) of compound of formula (1) in polymorphic form A.

### Example 6: Preparation of toluene solvate of compound of Formula (1)

Under nitrogen protection, 20 g of compound of Formula (8) was mixed with 150 g of 2-methyl tetrahydrofurane (MeTHF) and 6.8g of diisopropyl ethylamine (DIPEA). The suspension was cooled down to (-11)-(-13) °C and a solution of 4.7 g of acryloyl chloride in 50g of MeTHF was slowly added dropwise (in the course of 1 hour). The suspension was stirred at -12 °C next 10 minutes. The mixture was filtered at 10 - 15° C and the filter cake was washed with 2x5g of MeTHF .

The filtrate was combined with 0.30g of HCl (35-36%) at 25° C. The mixture was heated to 45° C and combined with 4g of active carbon. The mixture was heated to 50° C and stirred for 0.5 hour. The mixture was filtered at 40° C. The filtrate was concentrated under low pressure (40kPa, bath 20 - 22° C, 0.5hr) to provide 27.18 g of solid mass in a form of a foam.

The solid was dissolved in a mixture of MeTHF/toluene (1:2.5:5.0, solid:MeTHF:toluene, wt:wt:wt) at 55 - 60° C. The solution was filtered and cooled to 0 - 5°C to form suspension. The solid mass was filtered off, washed with toluene and dried to provide 22.55g (yield 82%) of toluene solvate of compound of Formula (1).

### Example 7: Preparation of polymorphic form A of compound of Formula (1)

22.55 g of toluene solvate of compound of Formula (1) were mixed with a mixture of 11g of MeTHF and 88g of methanol. The mixture was heated to 45°C. The mixture was cooled to 25° C during 0.5 hour, then solution was stirred at 25° C for 3hours. The suspension was cooled to 5 - 0° C and stirred for next 2hours. Obtained solid was filtered off, washed with 2x5g of cold (0°C) methanol and dried on filter for 0.5 hour then at room temperature (20-25°C) for 1 hour to provide 14.52 g (yield 63.7%) of compound of Formula (1) in crystalline form C. Obtained solid was mixed with 73g of methyl tert-butyl ether (MTBE) under nitrogen atmosphere and the mixture was stirred at 40°C for 2 hours. The suspension was cooled to room temperature (20-25°C) and obtained solid mass was filtered off, washed with 2x10g of MTBE and dried on filter for 0.5 hour to provide 14.2g (yield 97.8%) of solid compound of Formula (1) in polymorph form A.

## Claims

1. A process for preparation of Ibrutinib, compound of Formula 1, or a salt thereof, 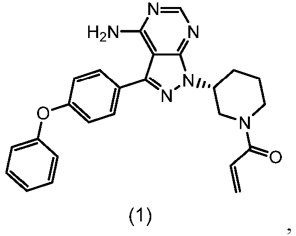 the process comprising:
a. Reacting compound of formula (2) with compound of formula (3) in a presence of Lewis acid to provide compound of Formula (4), 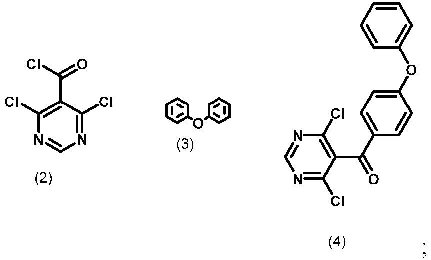
b. Contacting compound of Formula (4) with toluene;
c. Isolate a solid form of compound of formula (4) from toluene;
d. Transforming compound of Formula (4) into Ibrutinib.

2. The process according to claim 1 wherein the step c. comprises evaporating the mixture of compound of Formula (4) with toluene to between 45% and 55% of the original volume.

3. The process according to claims 1 or 2 wherein the solid form of compound of Formula (4), Form 1, is **characterized by** XRPD pattern having 2θ values 14.1°, 17.2°, 20.5° and 22.3° degrees 2 theta (± 0.2 degrees 2 theta) when measured with CuKα1 radiation (λ = 1.5406 Å).

4. The process according to anyone of claims 1 to 3 wherein step d. comprises:
i. Reacting compound of Formula (4) with ammonia to obtain a mixture;
ii. Reacting the mixture with hydrazine to obtain compound of Formula (5),

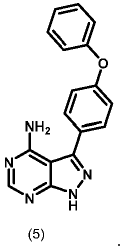

5. The process according to claim 4 wherein ammonia is aqueous solution of ammonia.

6. The process according to claim 4 wherein the hydrazine is hydrazine hydrate.

7. The process according to claim 4 further comprising:
i. Reacting compound of Formula (5) with compound of Formula (6) in a suitable solvent to provide compound of Formula (7), 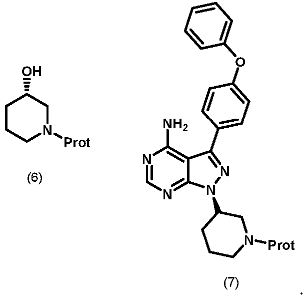 Prot is an amine protective group;
ii. Deprotecting compound of Formula (7) to provide compound of Formula (8) or a salt thereof, 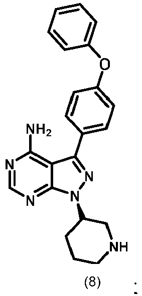
iii. transforming compound of Formula (8) or a salt thereof into Ibrutinib.

8. The process according to claim 7 wherein the suitable solvent is 2-methyl tetrahydrofurane.

9. The process according to claim 7 or 8 wherein the step iii. comprises:
i. Reacting of compound of Formula (8) with acryloyl chloride in 2-methyl tetrahydrofurane;
ii. Filtering the mixture;
iii. Adding an acid into the filtrate;
iv. Carbofiltration of the filtrate;
v. Isolating Ibrutinib.

10. The process according to claim 9 wherein the weight ratio between the acid and the compound of Formula (8) is between 1:63 and 1:69.

11. The process according to claim 9 or 10 wherein the acid is hydrochloric acid.

## Patentansprüche

1. Verfahren zur Herstellung von Ibrutinib, einer Verbindung der Formel 1, oder eines Salzes davon, 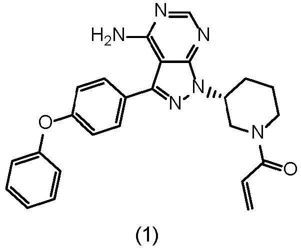 wobei das Verfahren umfasst:
a. Umsetzen einer Verbindung der Formel (2) mit einer Verbindung der Formel (3) in Gegenwart einer Lewis-Säure, um eine Verbindung der Formel (4) bereitzustellen, 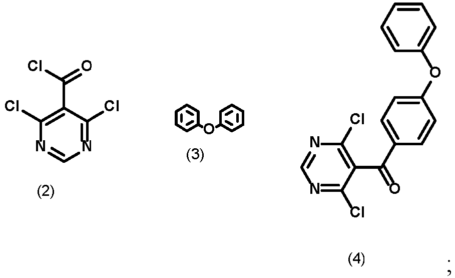
b. In-Kontakt-Bringen einer Verbindung der Formel (4) mit Toluol;
c. Isolieren einer festen Form der Verbindung der Formel (4) aus Toluol;
d. Umwandeln der Verbindung der Formel (4) zu Ibrutinib.

2. Verfahren nach Anspruch 1, wobei der Schritt c. Evaporieren des Gemisches der Verbindung der Formel (4) mit Toluol auf zwischen 45% und 55% des ursprünglichen Volumens umfasst.

3. Verfahren nach Ansprüchen 1 oder 2, wobei die feste Form der Verbindung der Formel (4), Form 1, durch ein XRPD-Muster mit 2θ-Werten von 14,1°, 17,2°, 20,5° und 22,3° Grad 2-Theta (± 0,2° Grad 2-Theta) beim Messen mit CuKα1-Strahlung (λ = 1,5406 Å) gekennzeichnet ist.

4. Verfahren nach einem beliebigen der Ansprüche 1 bis 3, wobei Schritt d. umfasst:
i. Umsetzen einer Verbindung der Formel (4) mit Ammoniak, um ein Gemisch zu erhalten,
ii. Umsetzen des Gemisches mit Hydrazin, um eine Verbindung der Formel (5) zu erhalten,

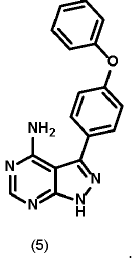

5. Verfahren nach Anspruch 4, wobei Ammoniak eine wässrige Ammoniaklösung ist.

6. Verfahren nach Anspruch 4, wobei das Hydrazin Hydrazinhydrat ist.

7. Verfahren nach Anspruch 4, das des Weiteren umfasst:
i. Umsetzen einer Verbindung der Formel (5) mit einer Verbindung der Formel (6) in einem geeigneten Lösungsmittel, um eine Verbindung der Formel (7) bereitzustellen, 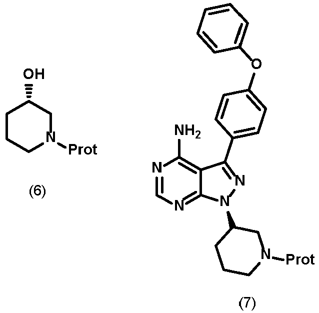 Prot ist eine Aminschutzgruppe;
ii. Entschützen der Verbindung der Formel (7), um eine Verbindung der Formel (8) oder ein Salz davon bereitzustellen; 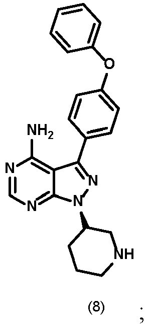
iii. Umwandeln einer Verbindung der Formel (8) oder eines Salzes davon zu Ibrutinib.

8. Verfahren nach Anspruch 7, wobei das geeignete Lösungsmittel 2-Methyltetrahydrofuran ist.

9. Verfahren nach Anspruch 7 oder 8, wobei der Schritt iii. umfasst:
i. Umsetzen einer Verbindung der Formel (8) mit Acryloylchlorid in 2-Methyl-tetrahydrofuran;
ii. Filtrieren des Gemisches;
iii. Zugeben einer Säure zum Filtrat;
iv. Kohlefiltration des Filtrats;
v. Isolieren von Ibrutinib.

10. Verfahren nach Anspruch 9, wobei das Gewichtsverhältnis zwischen der Säure und der Verbindung der Formel (8) zwischen 1:63 und 1:69 ist.

11. Verfahren nach Anspruch 9 oder 10, wobei die Säure Salzsäure ist.

## Revendications

1. Un procédé de préparation d'ibrutinib, le composé de formule 1, ou d'un sel en dérivant 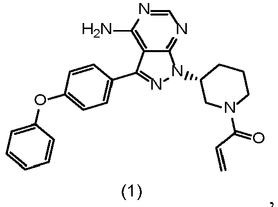 le procédé comprenant :
a) la réaction d'un composé de formule (2) avec un composé de formule (3) en présence d'un acide de Lewis pour obtenir un composé de formule (4), 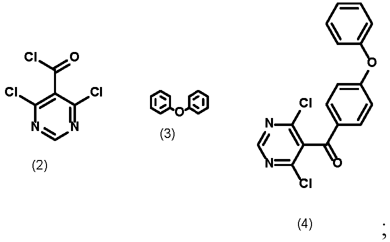
b) le contact du composé de formule (4) avec du toluène ;
c) la séparation entre le toluène et une forme solide d'un composé de formule (4);
d) la transformation d'un composé de formule (4) en ibrutinib.

2. Le procédé selon la revendication 1, dans lequel l'étape c. comprend l'évaporation du mélange du composé de formule (4) avec du toluène entre 45 % et 55 % du volume d'origine.

3. Le procédé selon les revendications 1 ou 2, dans lequel la forme solide du composé de formule (4), la forme 1, est **caractérisée par** un spectre XRPD présentant les valeurs 2θ de 14,1°, 17,2°, 20,5° et 22,3° degrés 2θ (± 0,2 degrés 2 thêta) lorsque mesurées avec le rayonnement CuKα1 (λ = 1,5406 Å).

4. Le procédé selon l'une quelconque des revendications 1 à 3, dans lequel l'étape d. comprend :
i. la réaction d'un composé de formule (4) avec de l'ammoniac pour obtenir un mélange ;
ii. la réaction dudit mélange avec de l'hydrazine pour obtenir le composé de formule (5),

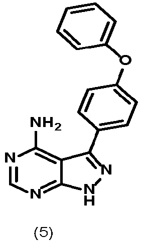

5. Le procédé selon la revendication 4, dans lequel l'ammoniac est une solution aqueuse d'ammoniac.

6. Le procédé selon la revendication 4, dans lequel l'hydrazine est de l'hydrate d'hydrazine.

7. Le procédé selon la revendication 4, comprenant en outre:
i. la réaction du composé de formule (5) avec le composé de formule (6) dans un solvant approprié pour obtenir un composé de formule (7), 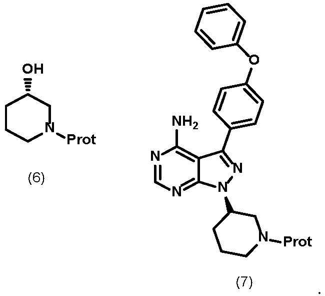 Prot est un groupe protecteur aminé;
ii. la déprotection du composé de formule (7) pour obtenir un composé de formule (8) ou d'un sel en dérivant. 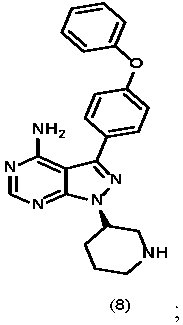
iii. la transformation en ibrutinib du composé de formule (8) ou d'un sel en dérivant.

8. Le procédé selon la revendication 7, dans lequel le solvant approprié est le 2-méthyltétrahydrofurane.

9. Le procédé selon la revendication 7 ou 8, dans lequel l'étape iii. comprend:
i. la réaction d'un composé de formule (8) avec du chlorure d'acryloyle dans du 2-méthyltétrahydrofurane ;
ii. la filtration du mélange obtenu;
iii. l'ajout d'un acide au filtrat;
iv. la carbofiltration du filtrat;
v. l'isolement de l'ibrutinib.

10. Le procédé selon la revendication 9, dans lequel le rapport pondéral de l'acide au composé de formule (8) est compris entre 1/63 et 1/69.

11. Le procédé selon la revendication 9 ou 10, dans lequel l'acide est de l'acide chlorhydrique.
